# EUROPEAN PATENT APPLICATION

(11) **EP 4 328 929 A1**
(43) Date of publication of application: **28.02.2024**
(21) Application number: 22791975.0
(22) Date of filing: 18.04.2022
(51) Int. Cl.: G16H 30/40, G06T 5/00, A61B 6/00, A61B 6/14, A61B 34/10, A61C 7/00

(54) **METHOD AND DEVICE FOR PROVIDING MEDICAL IMAGES FOR IDENTIFYING REGIONS OF INTEREST**

(30) Priority: 23.04.2021 KR 20210053091
(71) Applicant: Osstemimplant Co., Ltd., Gangseo-gu Seoul 07789 (KR)
(72) Inventor: JOO, Won Hyeong, Incheon 22697 (KR); PARK, Su Hee, Paju-si Gyeonggi-do 10908 (KR); CHOI, Kyoo OK, Seoul 07789 (KR)
(74) Representative: Berggren Oy
(86) International application number: PCT/KR2022/005535
(87) International publication number: WO 2022/225275

(57) **Abstract**

According to the present invention, disclosed are a method and device for providing medical images, in which: a list of conditions for providing medical images is displayed; a selection input for at least one medical image provision condition is received from the list by a user; and medical images are generated on the basis of the selection input, wherein pre-stored medical images are adjusted according to the medical image provision condition.

## Description

### Technical Field

The present invention relates to a method and apparatus for providing medical images to support to selectively identify a region of interest in a three-dimensional (3D) image.

### Background Art

Currently, in dental three-dimensional (3D) software (SW), a 3D image after computerized tomography (CT) scan may be provided in a volume rendering method. The method may simply show the shape of a captured target and although the user may identify the overall structure, it may be difficult to view each structure based on the purpose.

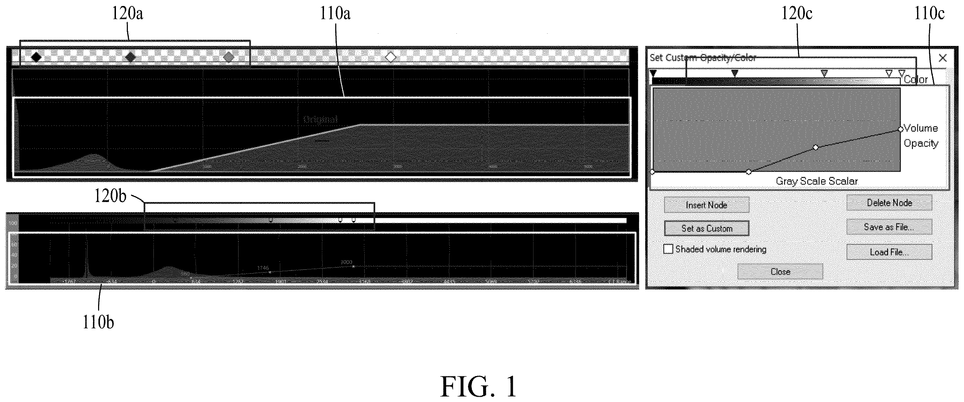

More particularly, as shown in FIG. 1, when the user desires to view a structure, such as a dental root, maxillary sinus, and the like, required to be checked, a volume rendering value may need to be set. However, a screen for setting most of volume rendering values may display house unit field (HU) values 110a, 110b, and 110c in a graph and the user may need to manually adjust values, such as the width and the height of the graph.

In addition, when the user desires to change a color, the user may need to click icons 120a, 120b, and 120c on the graph and change the color, and thus, it may be difficult to actually make adjustments unless the user has professional knowledge on the image.

Moreover, in most individual dental clinics, such knowledge may not be easily obtained and for an elderly user, it may be difficult to use.

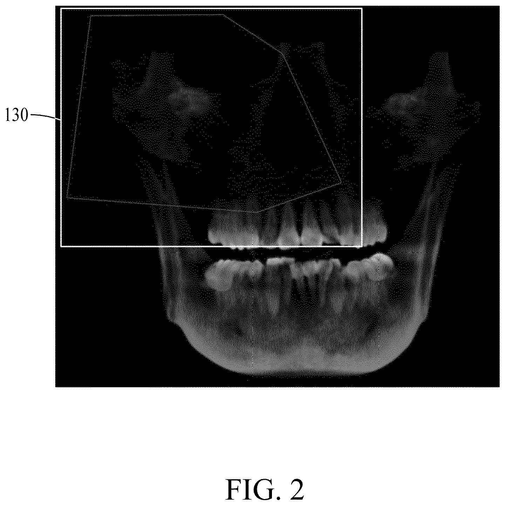

In addition, as shown in FIG. 2, when the user desires to identify only a predetermined portion of an image, the user may need to crop the image using a tool 130 referred to as Sculpt.

When some structures are distinguished through segmentation, the user may find use thereof to be easy, however, in most cases, Object provides only Show/Hide features.

Accordingly, to generate an image that the user desires to view through Show/Hide, the user may need to fit through structure Show/Hide tasks with multiple clicks and for adjusting the opacity, because it is formed as a slide bar, detailed adjustment may be required.

That is, currently, various processes and efforts may be required to select and view an image desired by the user.

### Disclosure of the Invention

### Technical Goals

The present invention is for solving a problem that multiple tasks are performed through editing or setting for a user to selectively identify a region of interest (ROI) in a 3D image. The purpose of the present invention is to change a configuration method of a structure to be identified through a medical image by a single click of the user.

In addition, another purpose is to provide a medical image to a user without editing.

In addition, another purpose is to easily perform image setting for a user.

Other purposes that are not specified in the present invention may be additionally considered in a scope that may be easily inferred from the following detailed descriptions and effects.

### Technical Solutions

To solve the above problem, a medical image providing method, performed by a medical image providing apparatus, according to an aspect of the present invention includes displaying a list of medical image providing conditions, receiving, from a user, a selection input related to at least one medical image providing condition from the list, generating an adjusted medical image in which a pre-stored medical image is adjusted based on a medical image providing condition based on the selection input, and providing the adjusted medical image.

Here, the list of medical image providing conditions is displayed in a button format.

Here, the medical image providing condition includes a first condition on a structure that the user desires to identify on a medical image, the first condition includes a plurality of structures included in the medical image, and opacity and color of each of the structures are set in advance.

Here, the generating of the adjusted medical image includes generating an adjusted medical image by adjusting opacity and color of each corresponding structure in a pre-stored medical image based on preset opacity and color to the structure selected by the user from the first condition.

Here, each structure matches a plurality of structure groups and each group includes the each structure and other structures having relations to the each structure and is defined by a combination of different structures, and the generating of the adjusted medical image includes providing a plurality of structure groups matched to the structure selected by the user in the first condition, determining one structure group of the plurality of structure groups based on selection of the user, and adjusting attributes of structures included in the structure group to the preset value.

Here, the other structures having relations to the each structure are adjacent structures disposed adjacent to each other, and an attribute of an adjacent structure disposed adjacent to the structure selected by the user is differently displayed.

Here, the medical image providing condition includes a second condition on treatment to be performed by the user, and the second condition includes at least one of implant, endodontic treatment (endo), orthodontic treatment (ortho), and multiplanar reformation (MPR).

Here, the generating of the adjusted medical image includes, based on an image selection standard that is set by condition with respect to treatment selected by the user from the second condition, selecting images corresponding to the selected treatment from pre-stored medical images and generating an adjusted medical image.

Here, the medical image providing condition includes a third condition on a region that the user desires to identify on a medical image, and the third condition includes at least one of a tooth region, a bone region, a maxilla region, a mandible region, a sinus region, a nerve region, a tempromandibular joint (TMJ) region, an airway region, and a panorama region.

In addition, the method further includes receiving a change request input for changing a medical image providing condition from the user.

Here, the generating of the adjusted medical image includes, when generating the adjusted medical image, reflecting a changed medical image providing condition based on the change request input.

According to another aspect of the present invention, a medical image providing apparatus includes a display configured to display a list of medical image providing conditions, a user input unit configured to receive, from a user, a selection input related to at least one of medical image providing conditions from the list, and a controller configured to generate a medical image adjusted from a pre-stored medical image based on a medical image providing condition and provide the adjusted medical image to the display, based on the selection input.

Here, the list of medical image providing conditions is displayed in a button format.

Here, the medical image providing condition includes a first condition on a structure that the user desires to identify on a medical image, the first condition includes a plurality of structures included in the medical image, and opacity and color of each of the structures are set in advance.

Here, the controller is configured to adjust an attribute of a structure selected by the user from the first condition based on an attribute of a corresponding structure in a pre-stored medical image.

Here, each structure matches a plurality of structure groups and each group includes the each structure and other structures having relations to the each structure and is defined by a combination of different structures, and the controller is configured to provide a plurality of structure groups matched to the structure selected by the user in the first condition, determine one structure group of the plurality of structure groups based on selection of the user, and generate the adjusted medical image by adjusting opacities and colors of structures included in the determined structure group to preset values, respectively.

Here, the other structures having relations to the each structure are adjacent structures disposed adjacent to each other, and an attribute of an adjacent structure disposed adjacent to the structure selected by the user is differently displayed.

Here, the medical image providing condition includes a second condition on treatment to be performed by the user, and the second condition includes at least one of implant, endo, ortho, and MPR.

Here, the controller is configured to, based on an image selection standard that is set by condition with respect to treatment selected by the user from the second condition, select images corresponding to the selected treatment from pre-stored medical images and generate an adjusted medical image.

Here, the medical image providing condition includes a third condition on a region that the user desires to identify on a medical image, and the third condition includes at least one of a tooth region, a bone region, a maxilla region, a mandible region, a sinus region, a nerve region, a TMJ region, an airway region, and a panorama region.

Here, the user input unit is configured to receive a change request input for changing a medical image providing condition from the user.

Here, the controller is configured to, when generating the adjusted medical image, reflect a changed medical image providing condition based on the change request input.

### Effects

As described above, according to example embodiments of the present invention, a user may change a configuration method of a structure to be identified through a medical image by a single click of the user.

In addition, a medical image may be provided without editing based on the treatment purpose of the user.

In addition, the user may easily perform image setting.

Although an effect is not explicitly stated herein, expected effects from technical features of the present invention and provisional effects are considered as described herein.

### Brief Description of Drawings

FIG. 1 is a diagram illustrating an example of a screen of a conventional medical image providing apparatus.
FIG. 2 is a diagram illustrating another example of a screen of a conventional medical image providing apparatus.
FIG. 3 is a block diagram of a medical image providing apparatus according to an example embodiment of the present invention.
FIG. 4 is a flowchart illustrating a medical image providing method according to an example embodiment of the present invention.
FIGS. 5 and 6 are diagrams illustrating an example of a screen of a medical image providing apparatus according to an example embodiment of the present invention.
FIGS. 7 to 10 are diagrams illustrating an example of a screen that displays a medical image for each structure in a medical image providing apparatus according to an example embodiment of the present invention.
FIGS. 11 to 14 are diagrams illustrating an example of a screen that displays a medical image for each structure in a medical image providing apparatus according to an example embodiment of the present invention.
FIG. 15 is a diagram illustrating another example of a screen that displays a medical image for each structure in a medical image providing apparatus according to an example embodiment of the present invention.
FIGS. 16 to 18 are diagrams illustrating a screen that appears when selecting "View Bone" in a medical image providing image according to various example embodiments of the present invention.
FIGS. 19 to 21 are diagrams illustrating a screen that appears when selecting "sinus" from "View Maxilla" in a medical image providing apparatus according to various example embodiments of the present invention.
FIGS. 22 and 23 are diagrams illustrating a screen that appears when selecting "nerve" from "View Mandible" in a medical image providing apparatus according to various example embodiments of the present invention.
FIGS. 24 and 25 are diagrams illustrating an example of a screen providing a medical image based on the purpose of treatment in a medical image providing apparatus according to an example embodiment of the present invention.
FIGS. 26 to 29 are diagrams illustrating an example of a screen providing a medical image based on the purpose of treatment in a medical image providing apparatus according to various example embodiments of the present invention.
FIGS. 30 and 31 are diagrams illustrating an example of a screen for changing a medical image providing condition in a medical image providing apparatus according to an example embodiment of the present invention.

### Best Mode for Carrying Out the Invention

Hereinafter, a medical image providing method and apparatus related to the present invention are described in detail with reference to drawings. However, the present invention may be implemented in many different forms and is not limited to the embodiments described herein. In addition, to clearly describe the present invention, parts irrelevant to the description may be omitted and the same reference numeral of the drawings may indicate the same member.

Hereinafter, the suffixes "module" and "unit" for components used herein are given or used interchangeably by considering only the ease of description and do not have distinguishable meanings or roles.

The present invention relates to a medical image providing method and apparatus.

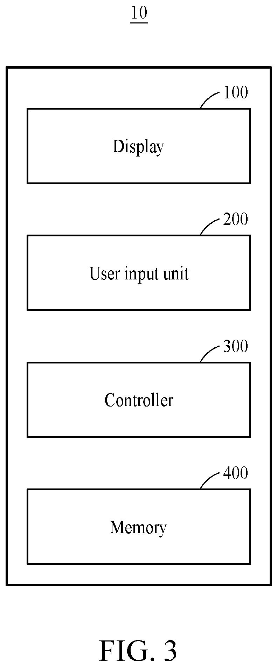

FIG. 3 is a block diagram of a medical image providing apparatus according to an example embodiment of the present invention.

Referring to FIG. 3, a medical image providing apparatus 10 according to an example embodiment of the present invention may include a display 100, a user input unit 200, a controller 300, and a memory 400.

The medical image providing apparatus 10 may be an apparatus for supporting a user to selectively identify a region of interest (ROI) in a three-dimensional (3D) image.

The medical image providing apparatus 10 according to an example embodiment of the present invention may change a configuration of a structure to be identified through a medical image by one input of a user.

The display 100 may display a list of medical image providing conditions.

The display 100 may display the list of medical image providing conditions and an adjusted medical image generated by the controller 300. In addition, information generated by the controller 300 may be displayed.

The user input unit 200 may receive a selection input related to at least one medical image providing condition from the list.

The user input unit 200 may be a means for inputting data for a user to control the medical image providing apparatus 10. The user input unit 200 may include a key pad, a dome switch, a touchpad (e.g., a contact capacitive type, a pressure resistive film type, an infrared sensing type, a surface ultrasonic conduction type, an integral tension measurement type, a piezo effect method, and the like), a jog wheel, a jog switch, or the like but is not limited thereto

Based on the selection input, the controller 300 may generate a medical image adjusted from a pre-stored medical image based on a medical image providing condition and may provide the adjusted medical image to a display.

The memory 400 may store the medical image.

In addition, the memory 400 may store programs (one or more instructions) for processing and controlling the controller 300, and the programs stored in the memory 400 may be divided into a plurality of modules based on their functions.

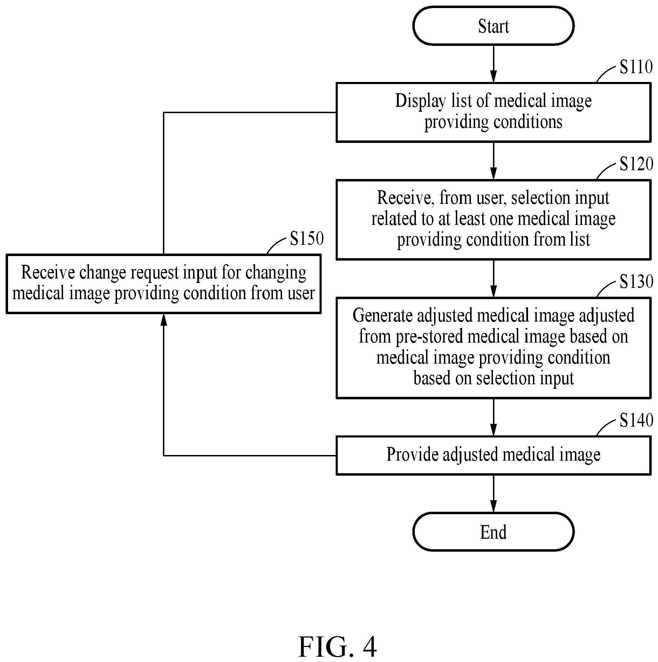

FIG. 4 is a flowchart illustrating a medical image providing method according to an example embodiment of the present invention.

Referring to FIG. 4, the medical image providing method according to an example embodiment of the present invention may be performed by a medical image providing apparatus and may begin with operation S110 of displaying a list of medical image providing conditions.

Thereafter, in operation S120, a selection input related to at least one medical image providing condition from the list may be received from a user.

In operation S130, based on the selection input, an adjusted medical image, in which a pre-stored medical image is adjusted based on the medical image providing condition, may be generated.

In operation S140, the adjusted medical image may be provided.

In addition, in operation S140, a change request input for changing a medical image providing condition may be received from the user and the list of medical image providing conditions may be changed.

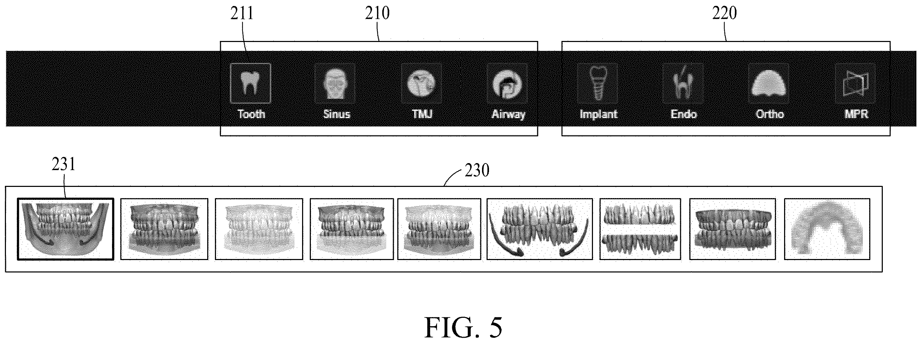

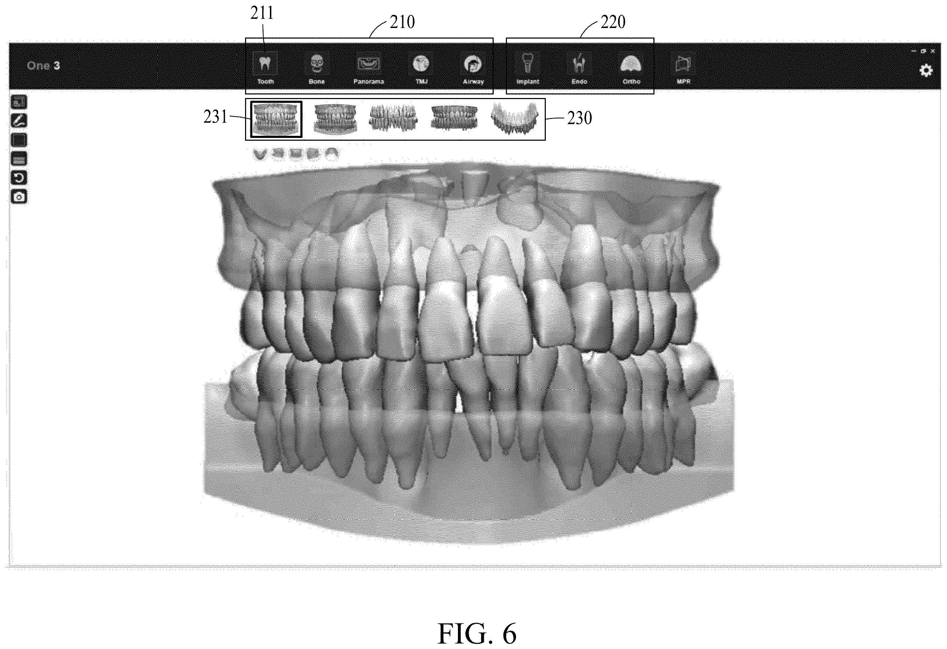

FIGS. 5 and 6 are diagrams illustrating an example of a screen of a medical image providing apparatus according to an example embodiment of the present invention.

The medical image providing apparatus according to an example embodiment of the present invention may perform a method of changing a configuration of a structure with a single click by providing preset image data in a button format and this may be referred to as a preset feature.

Referring to FIGS. 5 and 6, a list of medical image providing conditions may be displayed in a button format on a screen of the medical image providing apparatus according to various example embodiments of the present invention.

FIGS. 5 and 6 illustrate a software screen that varies depending on the version, and a user may variously change a configuration of the software screen.

In this case, the medical image providing conditions may include a first condition 230, a second condition 220, and a third condition 210.

The first condition 230 may be a condition on a structure that the user desires to identify on a medical image.

The first condition 230 may include a plurality of structures included in the medical image and opacity and color of each of the structures may be set in advance.

The second condition 220 may be a condition on treatment to be performed by the user.

The second condition 220 may include at least one of implant, endodontic treatment (endo), orthodontic treatment (ortho), and multiplanar reformation (MPR).

The third condition 210 may be a condition on a region that the user desires to identify on the medical image.

The third condition 210 may include at least one of a tooth region, a bone region, a maxilla region, a mandible region, a sinus region, a nerve region, a tempromandibular joint (TMJ) region, an airway region, and a panorama region.

For example, to identify a tooth region in a medical image, the user may identify the structure by clicking a button expressed as a tooth region icon 211 from the third condition 210 and then clicking a button 231 provided for each structure to be identified from the first condition 230.

However, at least one of the sinus region and the nerve region may not be individually expressed and may be expressed as a single structure included in a bone region. That is, in the bone region, the sinus region may be expressed as an empty region or the nerve region may be expressed as an empty region.

In addition, the bone region may be expressed as a structure divided into a maxilla region and a mandible region and may also be expressed as a more subdivided region, as necessary.

A screen in which a region of a structure is differently displayed based on the first condition 230 is described with reference to FIGS. 5 to 11.

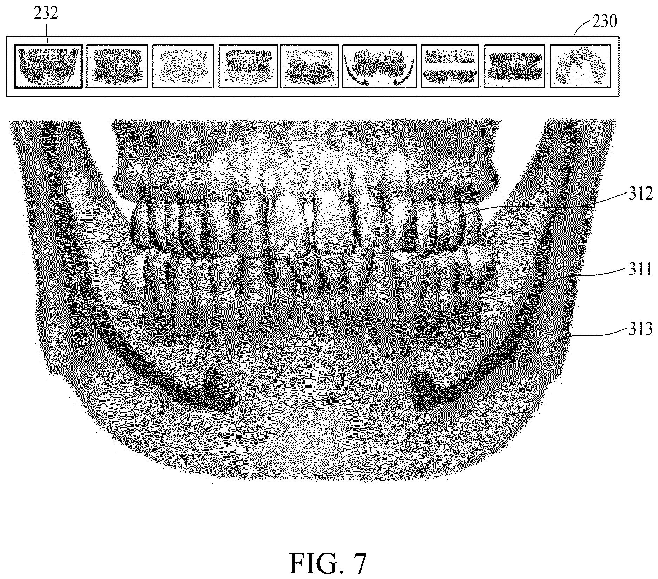

FIGS. 7 to 10 are diagrams illustrating an example of a screen that displays a medical image for each structure in a medical image providing apparatus according to an example embodiment of the present invention.

According to an example embodiment of the present invention, based on preset opacity and color of a structure selected by a user from the first condition, the medical image providing apparatus may adjust opacity and color of each corresponding structure in a pre-stored medical image and may generate the adjusted medical image.

Here, each structure may match a plurality of structure groups and each group may include each structure and other structures related to each structure and may be defined by a combination of different structures for each group.

In this case, for generating the adjusted medical image, the medical image providing apparatus may present a plurality of structure groups matched to a structure selected by the user from the first condition, may determine one structure group based on a user's selection among the plurality of structure groups, and may generate the adjusted medical image by adjusting opacities and colors of structures included in the determined structure group to preset values.

Here, the other structures related to each structure may be adjacent structures disposed adjacent to each other and in this case, the adjacent structure disposed adjacent to the structure selected by the user may be displayed in different opacity and color.

In a conventional case, an image may be provided based on a configuration of Tabs that the user desires to view, however, the user may need to identify adjacent structures together rather than simply viewing a main structure. To identify the adjacent structures together, various cases may arise from the perspective of the user.

Accordingly, cases for identifying only one adjacent structure, identifying two adjacent structures, or identifying an adjacent structure in a translucent state may be set in advance and an adjusted image may be provided to the user based on the preset standard.

For example, as shown in FIG. 7, when the user clicks a first button 232 of the first condition 230, the user may identify a structure including a neural tube 311, a tooth 312, and the jawbone 313.

In this case, since the neural tube 311, the tooth 312, and the jawbone 313 have high relations to each structure, the neural tube 311, the tooth 312, and the jawbone 313 may be set to be displayed together as adjacent structures even if only one of them is selected, and opacities and colors of the neural tube 311, the tooth 312, and the jawbone 313 may be differently displayed.

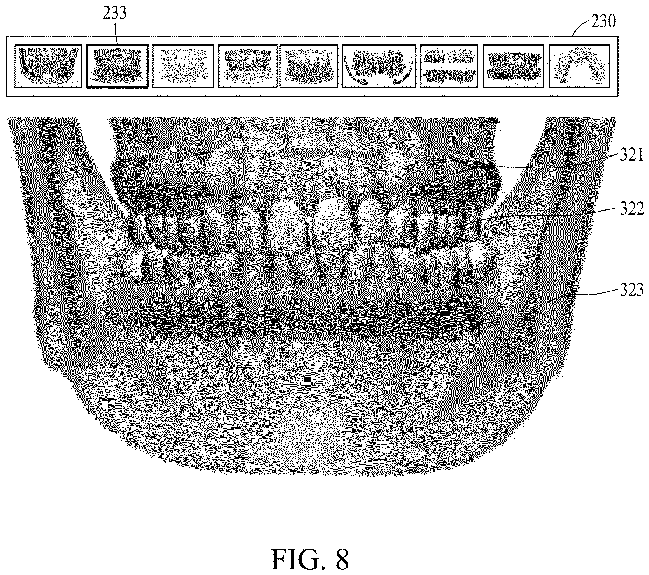

As shown in FIG. 8, when the user clicks a second button 233 of the first condition 230, the user may identify a structure including gum 321 and a tooth 322.

In this case, since the gum 321 and the tooth 322 have high relations to each structure, the gum 321 and the tooth 322 may be set to be displayed together as adjacent structures even if only one of them is selected, and attributes, such as opacities and colors, of the gum 321 and the tooth 322 may be differently displayed.

Since an adjacent structure having a high relation is set in advance for each structure and opacity and color of the adjacent structure is differently displayed, the user may identify an ROI without manually setting the adjacent structure and setting a different color and opacity thereof.

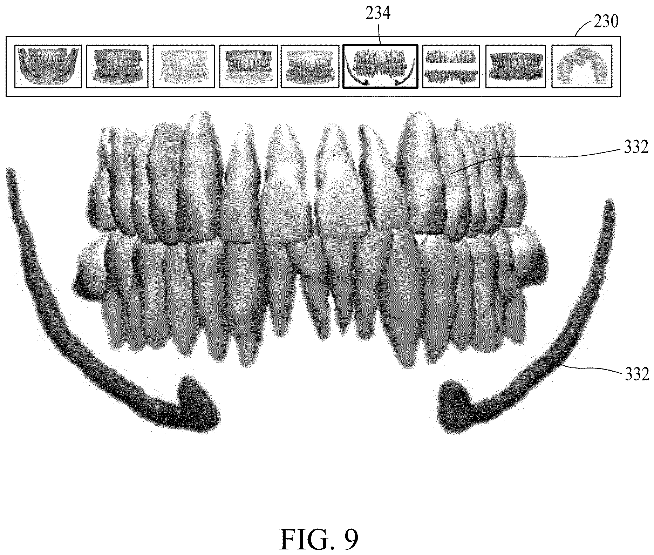

In addition, as shown in FIG. 9, when the user clicks a third button 234 of the first condition 230, the user may identify a structure including the neural tube 311 and the tooth 312.

In this case, unlike FIG. 7, the neural tube 311 and the tooth 312 may be more closely observed by excluding the jawbone and the neural tube 311 and the tooth 312 may be displayed in different opacities and colors.

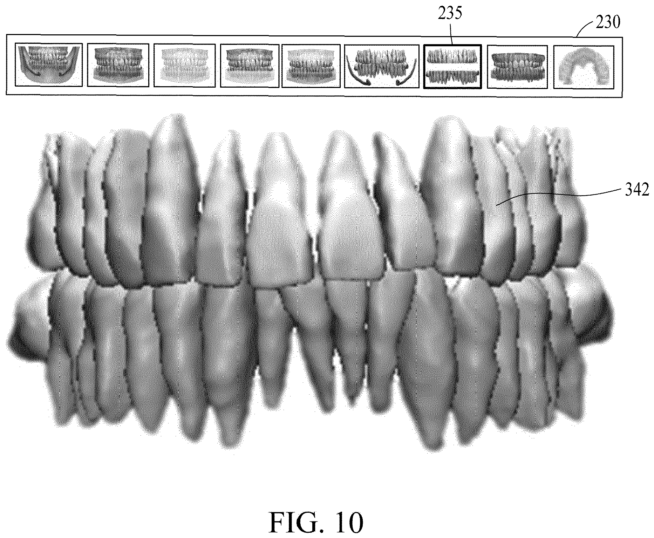

In addition, as shown in FIG. 10, when the user clicks a fourth button 235 of the first condition 230, the user may identify a tooth 342 structure.

Accordingly, the user may omit structure Show/Hide and opacity adjustment processes and may identify a desired image with a single click by changing and identifying an image by clicking a preset button.

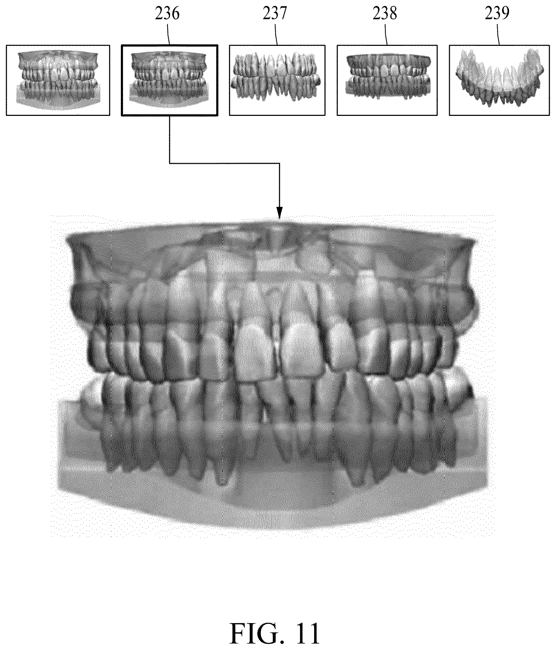

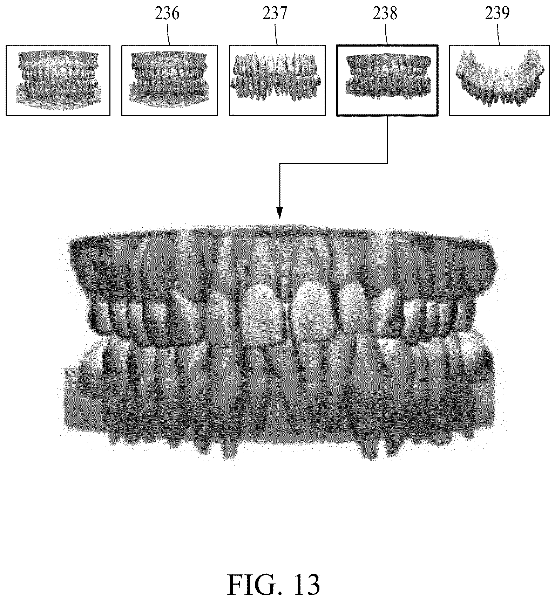

FIGS. 11 to 14 are diagrams illustrating an example of a screen that displays a medical image for each structure in a medical image providing apparatus according to various example embodiments of the present invention.

In a screen that displays a medical image by structure in a medical image providing apparatus according to various example embodiments of the present invention, as shown in FIG. 4B, a bone and a tooth may be identified.

In addition, as shown in FIG. 11, when clicking a fifth button 236, a bone, a tooth, and a gum structure may be identified.

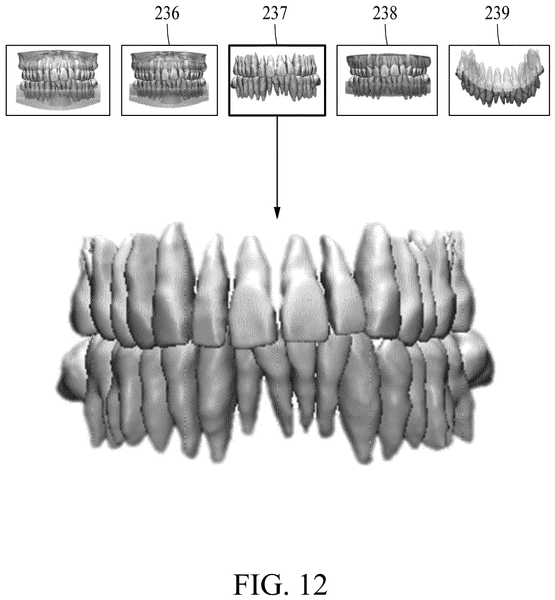

As shown in FIG. 12, when clicking a sixth button 237, a tooth structure may be identified.

As shown in FIG. 12, when clicking a seventh button 238, gum and a tooth structure may be identified.

Here, FIGS. 11 to 13 may provide the same structure as the structure included in FIGS. 5 and 6 and the design and configuration of the structure may be updated depending on the version.

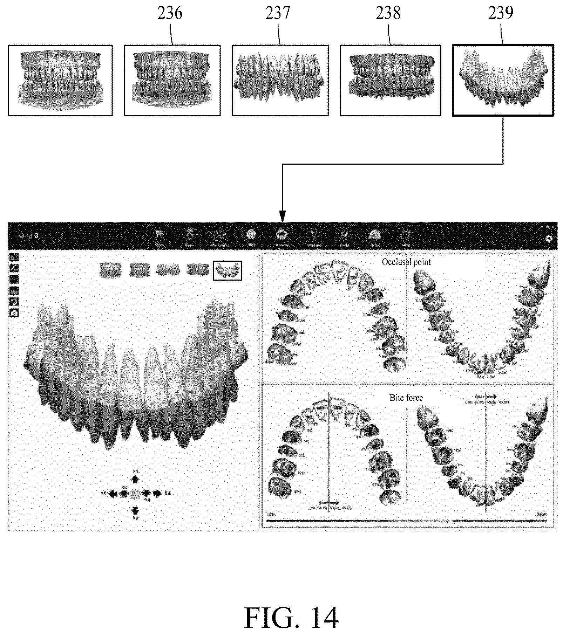

As shown in FIG. 14, when clicking an eighth button 239, an occlusal point and a bite force of a bone and a tooth may be identified.

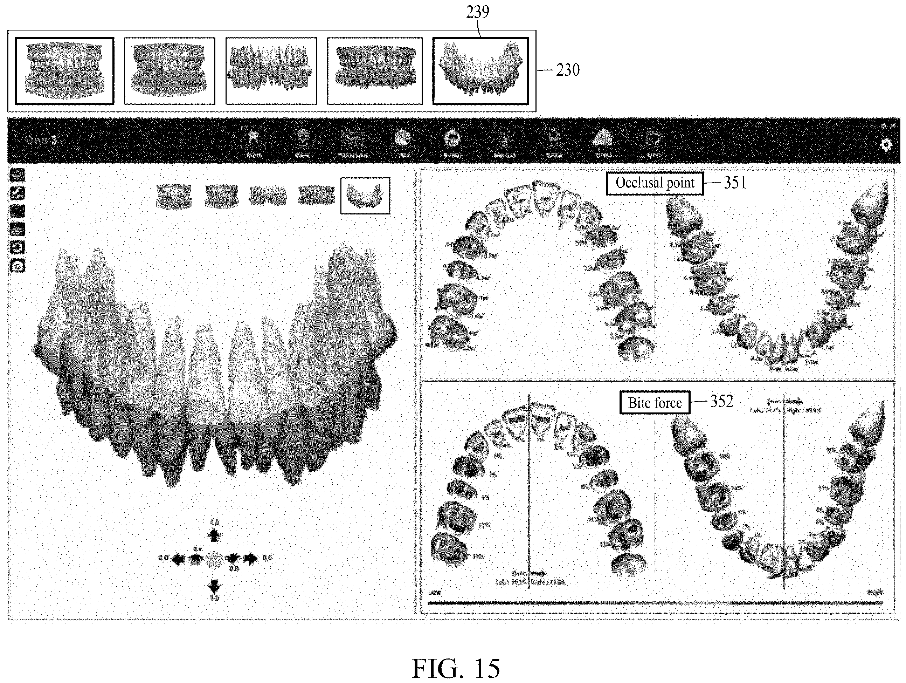

FIG. 15 illustrates a screen that appears when clicking the eighth button 239 from the first condition 230, as shown in FIG. 14.

As shown in FIG. 15, an image representing an occlusal point 351 with respect to a tooth and an image representing a bite force 352 may be identified.

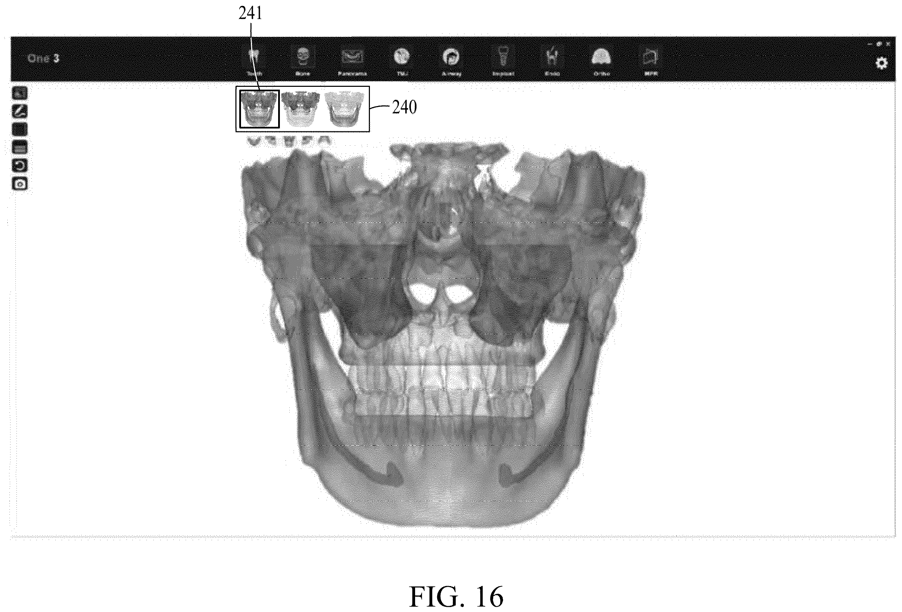

FIGS. 16 to 18 are diagrams illustrating a screen that appears when selecting "View Bone" in a medical image providing image according to various example embodiments of the present invention.

As shown in FIG. 16, when a user selects a bone region from the third condition 210 and then clicks a View maxilla, mandible 241 button from the first condition 240, the maxilla and mandible may simultaneously appear.

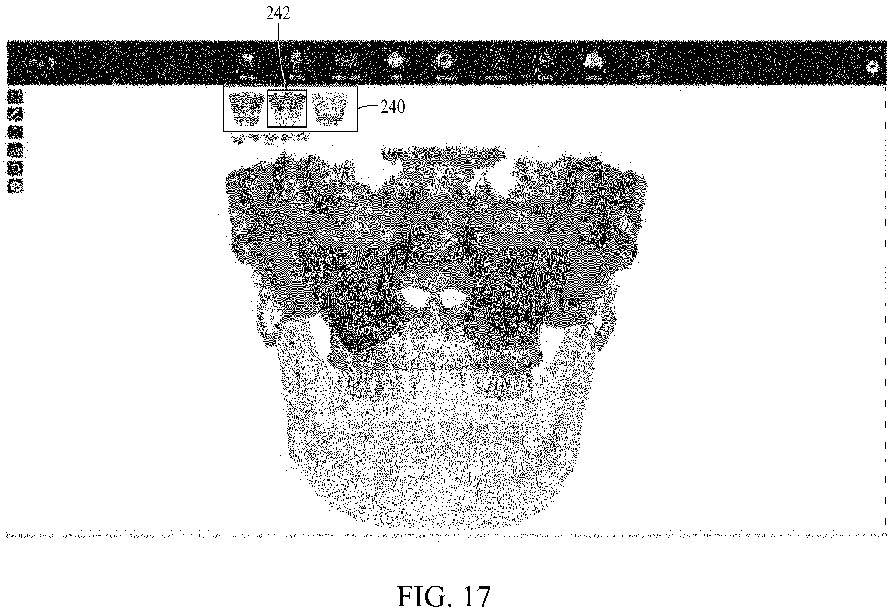

As shown in FIG. 17, when the user clicks a View maxilla 242 button from the first condition 240, the maxilla may appear and the opacity of the mandible may automatically decrease, and thus, the user may visually distinguish the maxilla and in this case, there is an advantage that the user may identify the mandible together.

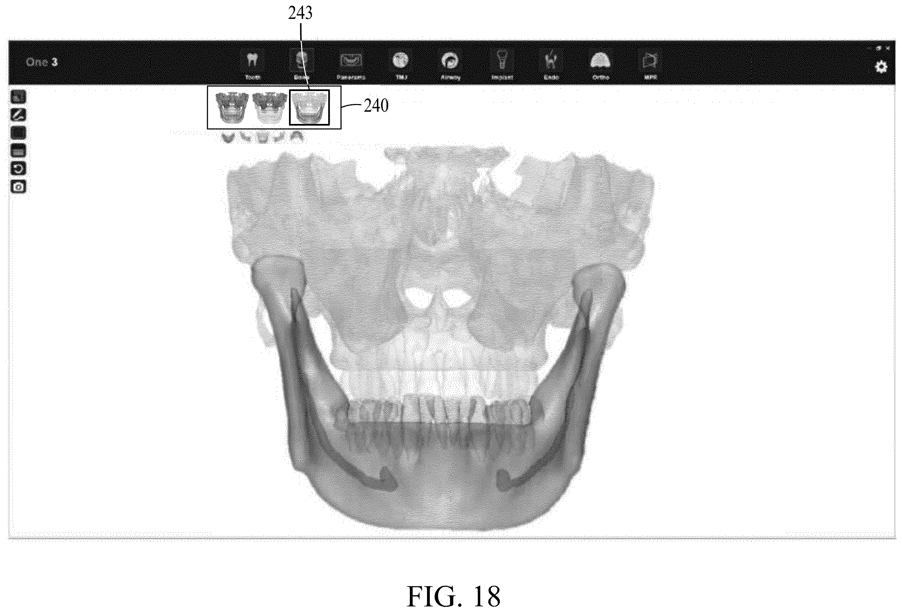

As shown in FIG. 18, when the user clicks a View mandible 243 button from the first condition 240, the mandible may appear and the opacity of the maxilla may automatically decrease, and thus, the user may visually distinguish the mandible and in this case, there is an advantage that the user may identify the maxilla together.

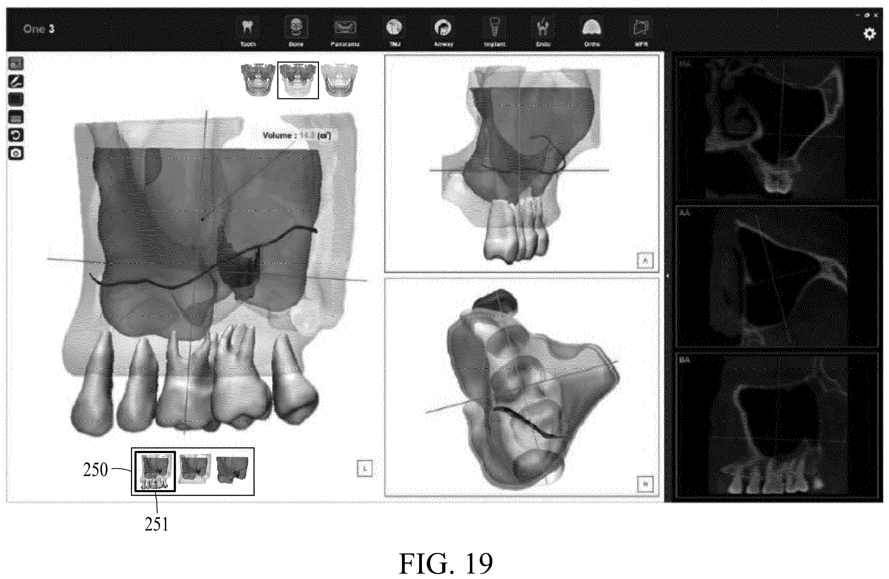

FIGS. 19 to 21 are diagrams illustrating a screen that appears when selecting "sinus" from "view maxilla" in a medical image providing apparatus according to various example embodiments of the present invention.

As shown in FIG. 19, when the user selects a sinus region from the third condition 210 and then clicks a View sinus bone, tooth 251 button from the first condition 250, a bone and tooth may simultaneously appear.

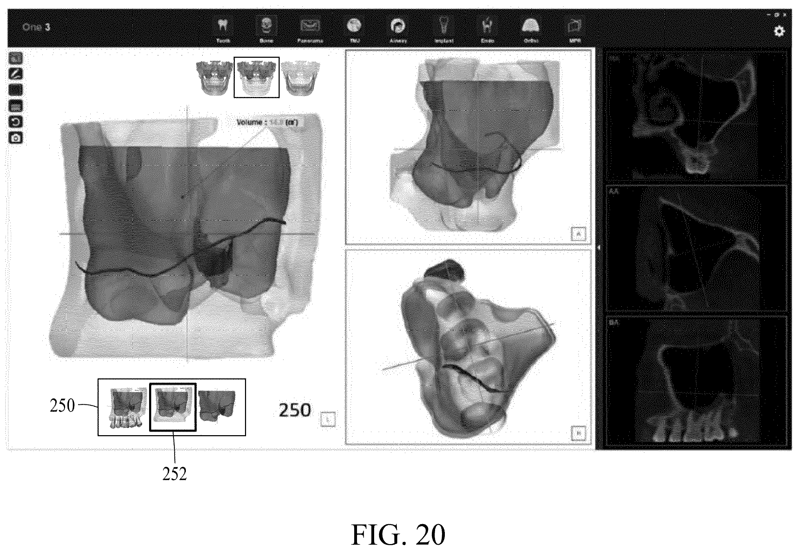

As shown in FIG. 20, when the user clicks a View sinus bone 252 button from the first condition 250, only the sinus bone may be displayed.

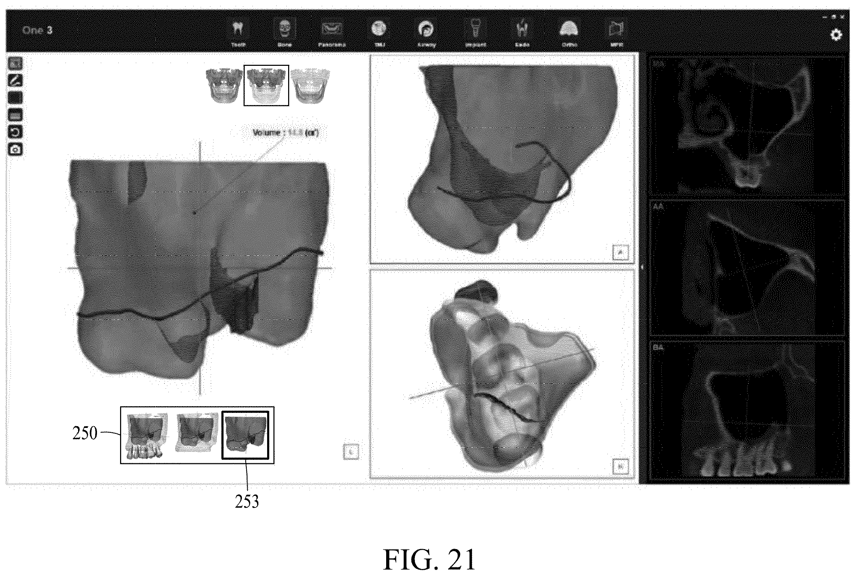

As shown in FIG. 21, when the user clicks a View sinus 253 button from the first condition 250, only the sinus may be displayed.

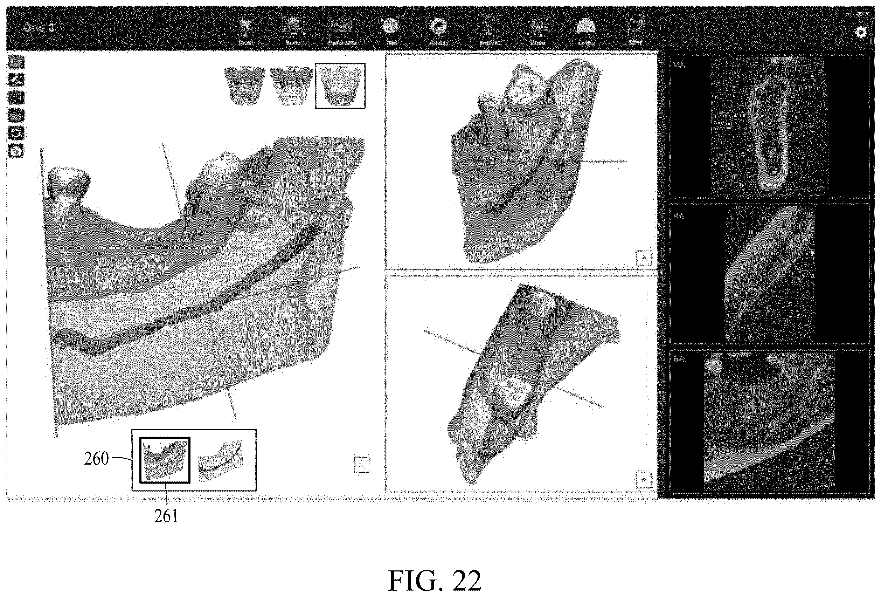

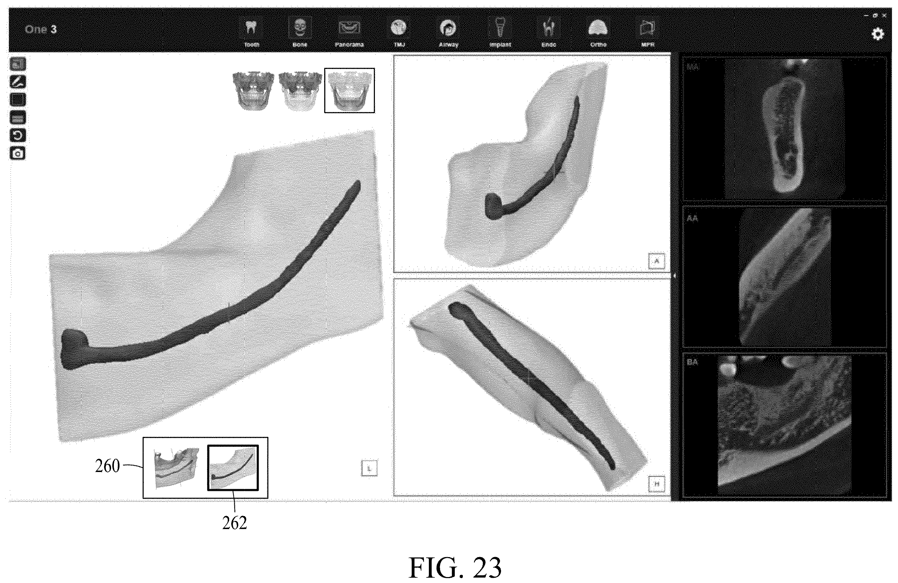

FIGS. 22 and 23 are diagrams illustrating a screen that appears when selecting "nerve" from "view mandible" in a medical image providing apparatus according to various example embodiments of the present invention.

As shown in FIG. 22, when a user clicks a nerve region from the third condition 210 and then clicks a View nerve bone, tooth 261 button from a first condition 260, the bone and tooth may simultaneously appear.

As shown in FIG. 23, when the user clicks a View nerve bone 262 button from the first condition 260, only the nerve bone may be displayed.

In addition, unlike FIG. 22, the opacity and color of the bone region may be automatically changed together.

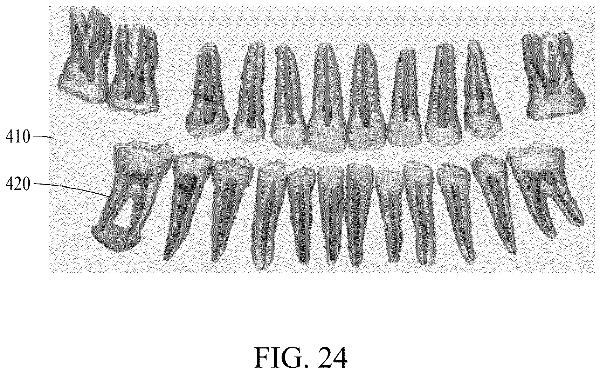

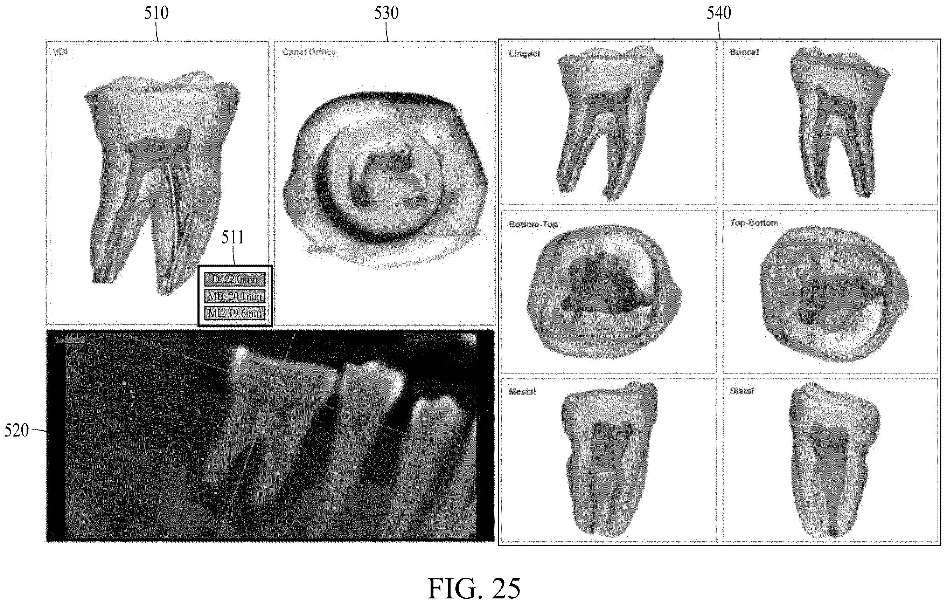

FIGS. 24 and 25 are diagrams illustrating an example of a screen providing a medical image based on the purpose of treatment in a medical image providing apparatus according to an example embodiment of the present invention.

The medical image providing apparatus according to an example embodiment of the present invention may perform a method of providing an optimized image as a main image based on the purpose of the treatment such that a user is able to use the image without further editing.

FIG. 24 may represent an endo panorama screen in which all the teeth and the overall dental pulp state may be identified and FIG. 25 may represent a screen in which dental pulp information of an individual tooth may be identified at a glance.

According to an example embodiment of the present invention, based on an image selection standard that is set by condition with respect to a treatment selected by the user from the second condition, the medical image providing apparatus may select images corresponding to the image selection standard from pre-stored medical images and may generate an adjusted medical image.

For example, because desired information when the user performs endo may be information of inflammation around the user, the shape of the dental pulp, a canal orifice of a tooth based on the overall shape of the teeth, and length information of the pulp, when the user clicks a button related to endo of the second condition 220, a screen related thereto may be set to be provided in advance and when the user clicks a button related to endo of the second condition 220, as shown in FIG. 24, an endo panorama screen 410 in which all the teeth and dental pulp states may be identified may be displayed.

In addition, when the user clicks a tooth 420 to identify the shape of the dental pulp together with the shape of the tooth in the endo panorama screen 410, as shown in FIG. 25, a screen, in which an individual tooth and dental pulp information may be identified at a glance, may be provided.

The user may identify VOI information 510 of the tooth, sagittal information 520, canal orifice information 530, and shape information 540 by angle and in the VOI information 510, dental pulp 511 of the tooth may be identified in detail.

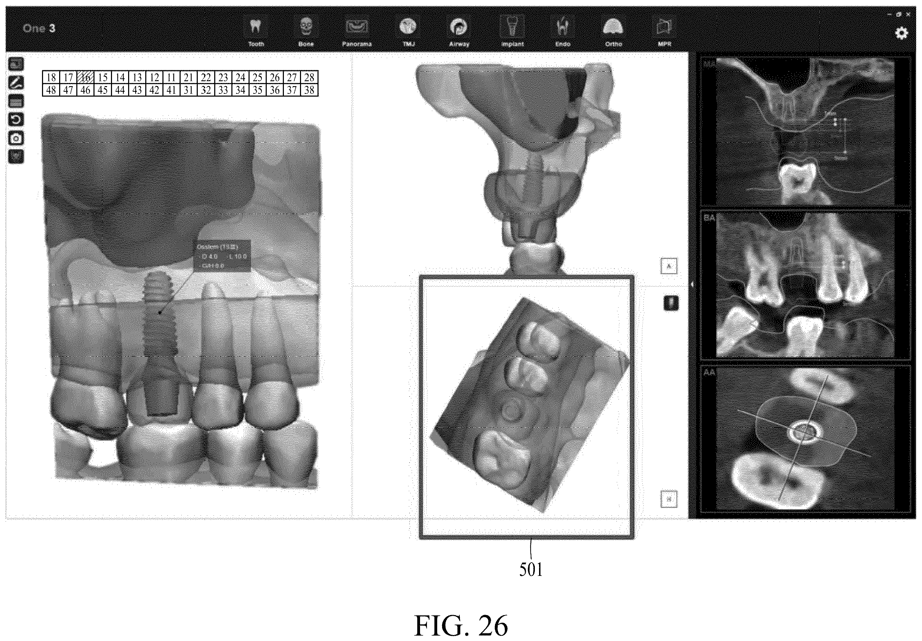

FIGS. 26 to 29 are diagrams illustrating an example of a screen providing a medical image based on the purpose of treatment in a medical image providing apparatus according to various example embodiments of the present invention.

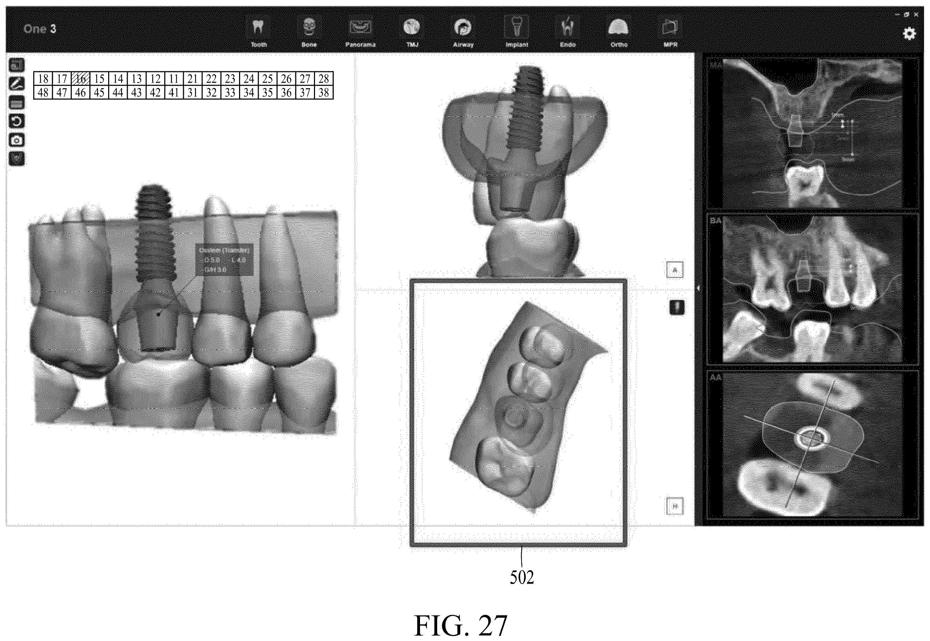

FIG. 26 may represent a case in which Fixture is selected from a screen that provides a medical image based on the purpose of treatment in a medical image providing apparatus according to various example embodiments of the present invention and FIG. 27 may represent a case in which Abutment is selected from a screen that provides a medical image based on the purpose of treatment in a medical image providing apparatus according to various example embodiments of the present invention.

According to various example embodiments of the present invention, preset may be automatically changed depending on a selected item and as shown in FIG. 26, a bone and an adjacent structure 501 may be identified when selecting Fixture, and as shown in FIG. 27, when selecting Abutment, a tooth and gum 502 may be identified.

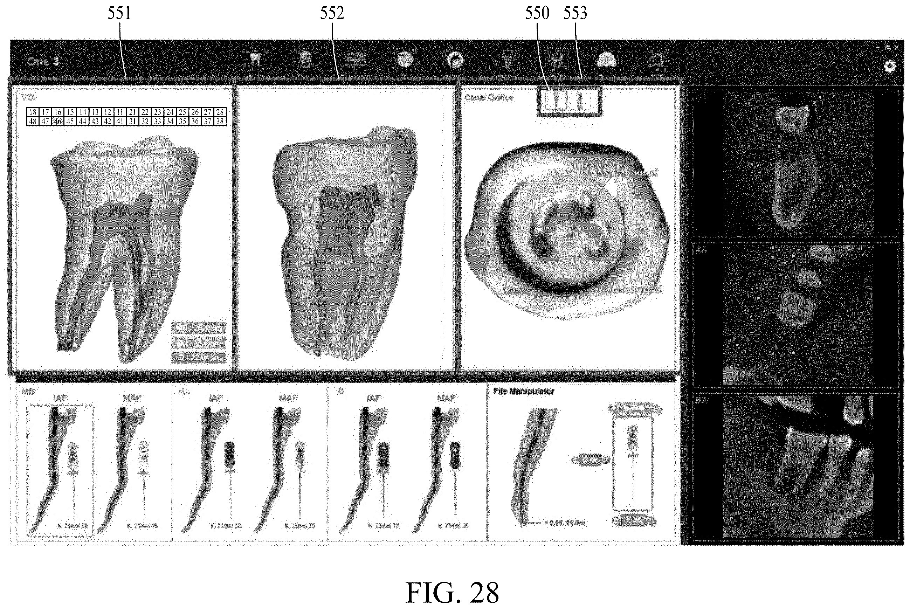

Referring to FIG. 28, when clicking a Check tooth 550 button in a screen that provides a medical image based on the purpose of treatment in a medical image providing apparatus according to various example embodiments of the present invention, an image (side) viewing in a Bucco-lingual axis (BA) direction 511 among tooth 3D models, an image (front) viewing in a Mesio-Distal axis (MA) direction among tooth 3D models, and a canal orifice (upper end) 553 among tooth 3D models may be identified.

Here, FIG. 28 may provide information, such as dental pulp information of a tooth included in FIG. 25, and the design and configuration of information provided depending on the version may be updated.

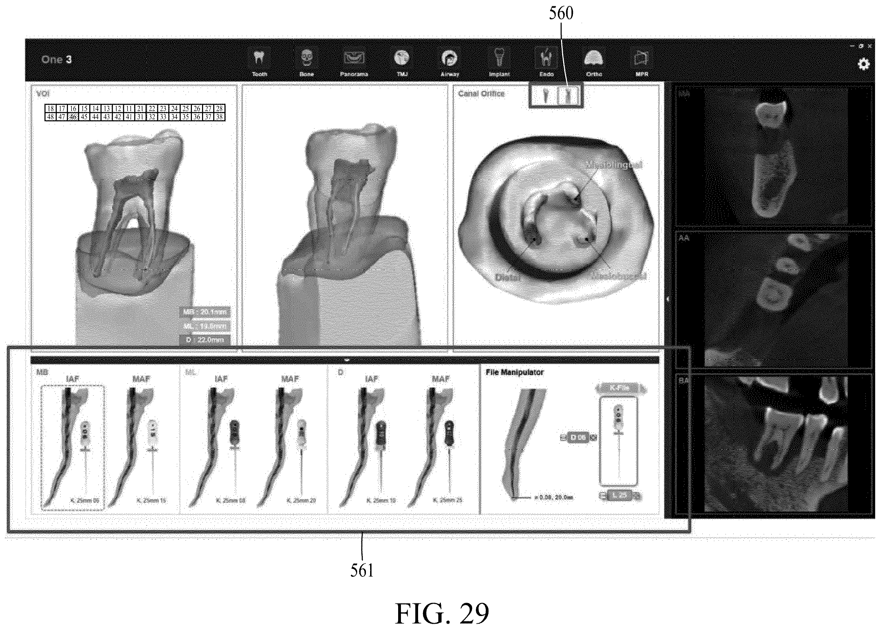

In addition, referring to FIG. 29, when clicking a Check treatment method 560 button, the user may identify a treatment method as a treatment method (sequence) 561 of a File Sequence file is displayed together.

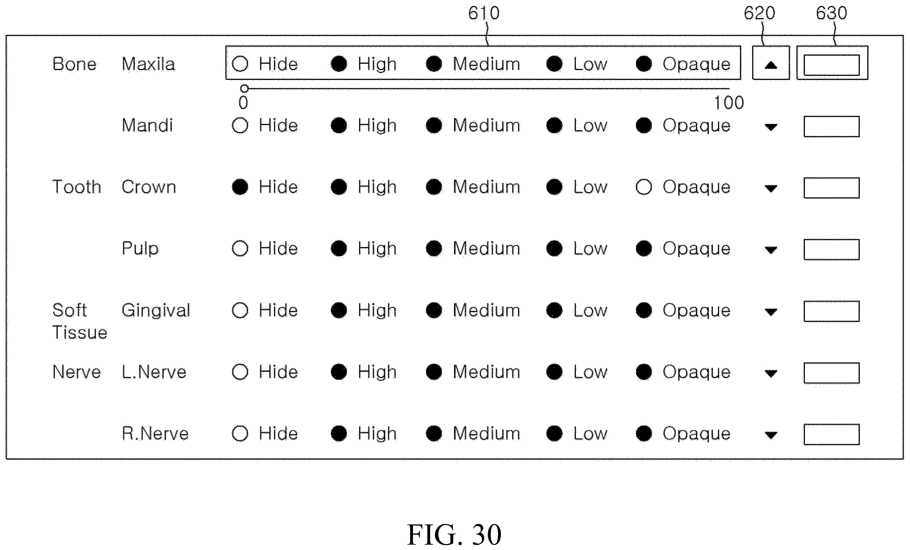

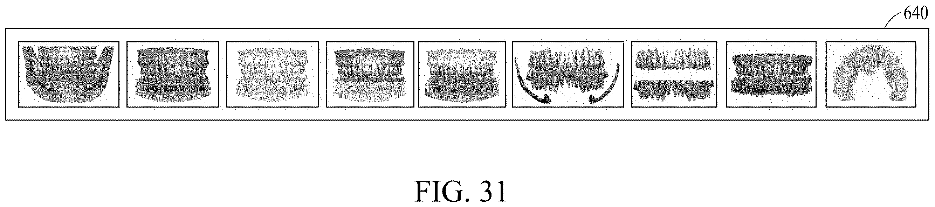

FIGS. 30 and 31 are diagrams illustrating an example of a screen for changing a medical image providing condition in a medical image providing apparatus according to an example embodiment of the present invention.

The medical image providing apparatus according to an example embodiment of the present invention may perform a method of assisting a user to easily set an image with 3 types of buttons rather than demanding the user to adjust detailed values by providing a scroll bar and consecutive values for setting the image.

According to an example embodiment of the present invention, the medical image providing apparatus may receive a change request input for changing a medical image providing condition from the user and when generating an adjusted medical image based on the change request input, the medical image providing apparatus may reflect a changed medical image providing condition.

As shown in FIG. 30, when the user changes opacity, the opacity may be changed with a single click, not continuously but discontinuously, by selecting an opacity change item 610 by bone, tooth, soft tissue, and nerve.

For example, a value of an opacity button in 3 stages may be set to Low: 25%, Medium: 50%, and High: 75%, and when the user clicks the button, the opacity may be automatically changed to the set value and when pressing a Preset button, the preset value that is set in advance may be identified.

For example, in the case of an image of a tooth and bone together, the tooth and the bone may be automatically selected to be 100% and 50%, respectively, and Medium may be selected for the bone. Here, when selecting Low, the bone may be more transparently viewed and when selecting High, the bone may be more clearly viewed.

If the user desires to continuously adjust the opacity, the user may set the opacity through an adjustment button 620 as a slide bar appears below.

In addition, by using a button 630 for setting a color by bone, tooth, soft tissue, and nerve, the user may set a color of the entire structure without adjusting colors for each region.

Accordingly, list information of medical image providing conditions displayed on a screen may be automatically changed and when generating an adjusted medical image, a changed medical image providing condition may be automatically reflected.

As shown in FIG. 3, because a preset value that is set in advance exists, opacity that is set based on a selected preset and a structure may be automatically changed and a default value that automatically changes may be set by the user.

Through the medical image providing apparatus according to an example embodiment of the present invention, a 3D viewer may be more easily used and an elderly user may be able to easily use and apply the latest software (SW) technology. Accordingly, by providing better image information to an elderly user, a user's state may be accurately identified and a patient may receive a more accurate and decent service as well as it may contribute to reduce medical accidents.

The above description is only an example embodiment of the present invention and one of ordinary skill in the art of the present invention may implement in a modified form without departing from the scope of the fundamental characteristic of the present invention. Therefore, the scope of the present invention is not limited to the aforementioned example embodiments and shall be interpreted to include various embodiments within the scope that is equivalent to the claims.

## Claims

1. A medical image providing method, performed by a medical image providing apparatus, comprising:
displaying a list of medical image providing conditions;
receiving, from a user, a selection input related to at least one medical image providing condition from the list;
generating an adjusted medical image in which a pre-stored medical image is adjusted based on a medical image providing condition based on the selection input; and
providing the adjusted medical image.

2. The medical image providing method of claim 1, wherein the medical image providing condition comprises a first condition on a structure that the user desires to identify on a medical image, and
the first condition comprises a plurality of structures comprised in the medical image.

3. The medical image providing method of claim 2, wherein the generating of the adjusted medical image comprises:
adjusting an attribute of a structure selected by the user from the first condition based on an attribute of a corresponding structure in a pre-stored medical image.

4. The medical image providing method of claim 2, wherein each structure matches a plurality of structure groups and each group comprises the each structure and other structures having relations to the each structure and is defined by a combination of different structures, and
the generating of the adjusted medical image comprises:
providing a plurality of structure groups matched to the structure selected by the user in the first condition;
determining one structure group of the plurality of structure groups based on selection of the user; and
adjusting attributes of structures comprised in the structure group to a preset value.

5. The medical image providing method of claim 4, wherein the other structures having relations to the each structure are adjacent structures disposed adjacent to each other, and
an attribute of an adjacent structure disposed adjacent to the structure selected by the user is differently displayed.

6. The medical image providing method of claim 1, wherein the medical image providing condition comprises a second condition on treatment to be performed by the user, and
the second condition comprises at least one of implant, endodontic treatment (endo), orthodontic treatment (ortho), and multiplanar reformation (MPR).

7. The medical image providing method of claim 6, wherein the generating of the adjusted medical image comprises:
based on an image selection standard that is set by condition with respect to treatment selected by the user from the second condition, selecting images corresponding to the selected treatment from pre-stored medical images and generating an adjusted medical image.

8. The medical image providing method of claim 1, wherein the medical image providing condition comprises a third condition on a region that the user desires to identify on a medical image, and
the third condition comprises at least one of a tooth region, a bone region, a maxilla region, a mandible region, a sinus region, a nerve region, a tempromandibular joint (TMJ) region, an airway region, and a panorama region.

9. The medical image providing method of claim 1, further comprising:
receiving a change request input for changing a medical image providing condition from the user.

10. The medical image providing method of claim 9, wherein the generating of the adjusted medical image comprises:
when generating the adjusted medical image, reflecting a changed medical image providing condition based on the change request input.

11. A medical image providing apparatus comprising:
a display configured to display a list of medical image providing conditions;
a user input unit configured to receive, from a user, a selection input related to at least one of medical image providing conditions from the list; and
a controller configured to generate a medical image adjusted from a pre-stored medical image based on a medical image providing condition and provide the adjusted medical image to the display, based on the selection input.

12. The medical image providing apparatus of claim 11, wherein the medical image providing condition comprises a first condition on a structure that the user desires to identify on a medical image, and
the first condition comprises a plurality of structures comprised in the medical image.

13. The medical image providing apparatus of claim 11, wherein the controller is configured to:
adjust an attribute of a structure selected by the user from the first condition based on an attribute of a corresponding structure in a pre-stored medical image.

14. The medical image providing apparatus of claim 12, wherein each structure matches a plurality of structure groups and each group comprises the each structure and other structures having relations to the each structure and is defined by a combination of different structures, and
the controller is configured to provide a plurality of structure groups matched to the structure selected by the user in the first condition, determine one structure group of the plurality of structure groups based on selection of the user, and generate the adjusted medical image by adjusting opacities and colors of structures comprised in the determined structure group to preset values, respectively.

15. The medical image providing apparatus of claim 14, wherein the other structures having relations to the each structure are adjacent structures disposed adjacent to each other, and
an attribute of an adjacent structure disposed adjacent to the structure selected by the user is differently displayed.

16. The medical image providing apparatus of claim 11, wherein the medical image providing condition comprises a second condition on treatment to be performed by the user, and
the second condition comprises at least one of implant, endodontic treatment (endo), orthodontic treatment (ortho), and multiplanar reformation (MPR).

17. The medical image providing apparatus of claim 16, wherein the controller is configured to:
based on an image selection standard that is set by condition with respect to treatment selected by the user from the second condition, select images corresponding to the selected treatment from pre-stored medical images and generate an adjusted medical image.

18. The medical image providing apparatus of claim 11, wherein the medical image providing condition comprises a third condition on a region that the user desires to identify on a medical image, and
the third condition comprises at least one of a tooth region, a bone region, a maxilla region, a mandible region, a sinus region, a nerve region, a tempromandibular joint (TMJ) region, an airway region, and a panorama region.

19. The medical image providing apparatus of claim 11, wherein the user input unit is configured to receive a change request input for changing a medical image providing condition from the user.

20. The medical image providing apparatus of claim 19, wherein the controller is configured to, when generating the adjusted medical image, reflect a changed medical image providing condition based on the change request input.
